(19) Europäisches Patentamt

European Patent Office

Office européen des brevets

(11) **EP 4 755 313 A1**

(12) **EUROPEAN PATENT APPLICATION**

(43) Date of publication:
**10.06.2026 Bulletin 2026/24**

(21) Application number: **25220500.0**

(22) Date of filing: **03.12.2025**

(51) International Patent Classification (IPC):
**A61B 8/08** *(2006.01)* **A61B 8/12** *(2006.01)*
**A61B 8/00** *(2006.01)*

(52) Cooperative Patent Classification (CPC):
**A61B 8/0883; A61B 8/12; A61B 8/445;
A61B 8/4461; A61B 8/4466; A61B 8/483;
A61B 8/5207; A61B 8/543;** A61B 8/4254;
A61B 8/4281; A61B 8/4494; A61B 8/52; A61B 8/56

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **04.12.2024 CN 202411764447**

(71) Applicant: **Shenzhen Cardioacc Ltd
Shenzhen 518000 (CN)**

(72) Inventors:
• **He, Jingcai
Shenzhen 518000 (CN)**
• **Zhou, Xinhuan
Shenzhen 518000 (CN)**

(74) Representative: **Metida
Gyneju str. 16
01109 Vilnius (LT)**

(54) **4D ICE CATHETER, ULTRASOUND IMAGING EQUIPMENT AND METHOD**

(57) A 4D intracardiac echocardiography catheter, equipment, and imaging method are provided. The catheter includes a sheath, an ultrasound transducer assembly, a bearing, and an external lumen. An internal lumen is provided inside the sheath. The ultrasound transducer assembly is rotatably connected to the sheath and is suspended in the internal lumen. The bearing is connected to the ultrasound transducer assembly and an external steering handle. The external lumen is connected to the bearing and is located on one side of the bearing that is away from the ultrasound transducer assembly. The catheter has a compact structure and high imaging quality.

FIG. 1

## Description

### TECHNICAL FIELD

The present disclosure relates to the technical field of ultrasound imaging equipment, and in particular, to a 4D intracardiac echocardiography (ICE) catheter, ultrasound imaging equipment and method.

### BACKGROUND

In cardiac minimally invasive interventional procedures, real-time visualization of cardiac structures directly affects intervention safety and efficiency. A traditional guiding method mainly relies on X-ray imaging, which has limited applications due to radiation, relatively low frame rate and restricted viewing angle. A safer, real-time, and more intuitive methods for observing cardiac structures is clinically valuable.

ICE technology provides a radiation-free method for observing the cardiac structures. The ICE technology embeds an ultrasound transducer at a distal end of the catheter to achieve real-time observation from inside of the cardiac structures. By adjusting the bending direction and angle of the ultrasound transducer, physicians can flexibly switch the imaging field of view and clearly see key information such as the structure, blood flow, and catheter position inside the heart.

Traditional ICE technology is typically 2D (two-dimensional) imaging with a compromised imaging field of view, while heart is beating fast in three-dimensional. 4D (four-dimensional, equivalent to real-time three-dimensional) ICE is a novel technology that provides 4D view of the heart, which makes intervention more intuitive and more efficient. However, current 4D ICE technology usually uses a complicated matrix array transducer integrated with a micro-beamforming ASIC, which contains a tremendous number of elements and is cost-prohibitive.

Therefore, it is necessary to propose a more cost-efficient 4D ICE method and apparatus to address these issues.

### SUMMARY

The present disclosure aims to provide a 4D ICE catheter, ultrasound imaging equipment and method for more accurate and more intuitive 4D imaging guidance of cardiac intervention.

The present disclosure is defined by the independent claims, which further define advantageous embodiments.

In the first aspect of the present disclosure, a 4D ICE catheter is recited in claim 1.

In the second aspect of the present disclosure, ultrasound imaging equipment is also provided as recited in claim 8.

In the third aspect of the present disclosure, an ultrasound imaging method, wherein the 4D ICE catheter according to any one of the aforementioned aspects is used, or the ultrasound imaging equipment according to any one of the aforementioned aspects is used, and the ultrasound imaging method provided as recited in claim 15 is disclosed.

Other aspects of the present disclosure are recited in the dependent claims attached hereto.

Implementation of the embodiments of the present disclosure has the following beneficial effects:

in the 4D ICE catheter of this embodiment, the rotatable ultrasound transducer assembly is configured to rotate within the external sheath. The ultrasound transducer assembly can be driven by a coil through a bearing, to achieve scanning in different rotation angles.

The ultrasound imaging equipment of this embodiment has two imaging modes: a 2D imaging mode and a 4D imaging mode. When the bearing acoustic housing rotates slowly or remains stationary, the ultrasound imaging equipment is in the 2D imaging mode; when the bearing rotates rapidly, the ultrasound imaging equipment is in the 4D imaging mode. When the transducer rotates slowly in the 2D mode, the ultrasound imaging equipment can be temporally synchronized with the electrocardiogram gating signal to perform image segmentation and 3D surface reconstruction on a series of 2D ultrasound images captured at different rotation angles, usually at the cardiac end-diastolic phase. In this way, physicians can obtain a 3D model of each specific cardiac structure at the cardiac end-diastolic phase. The image segmentation employs an artificial intelligence (AI)-based deep learning neural network method.

### BRIEF DESCRIPTION OF THE DRAWINGS

To describe the technical solutions in the embodiments of the present disclosure or in the related art more clearly, the following briefly introduces the accompanying drawings for describing the embodiments or the related art. Apparently, the accompanying drawings in the following description show merely some embodiments of the present disclosure, and a person of ordinary skill in the art may still derive other drawings from the accompanying drawings without creative efforts.

Where:

FIG. 1 shows a schematic cross-sectional structural diagram of a 4D ICE catheter in one embodiment of the present disclosure.

FIG. 2 shows a schematic cross-sectional structural diagram of a 4D ICE catheter in one embodiment of the present disclosure.

FIG. 3 shows a schematic structural diagram of ultrasound imaging equipment in an embodiment of the present disclosure.

FIG. 4 shows a schematic diagram of an imaging method of a 4D ICE catheter in one embodiment of the present disclosure.

FIG. 5 shows a flowchart of an ultrasound imaging method in one embodiment of the present disclosure.

FIG. 6 shows a schematic structural diagram of ultrasound imaging equipment in one embodiment of the present disclosure.

FIG. 7 shows another schematic structural diagram of ultrasound imaging equipment in one embodiment of the present disclosure.

FIG. 8 shows a schematic structural diagram of an ultrasound transducer in one embodiment of the present disclosure.

FIG. 9 shows a schematic diagram of imaging of a matrix array transducer in one embodiment of the present disclosure.

FIG. 10 shows a flowchart of an ultrasound imaging method in one embodiment of the present disclosure.

FIG. 11 shows a schematic diagram of a spherical coordinate system and a Cartier coordinate system in one embodiment of the present disclosure.

FIG. 12 shows structural block diagram of an ultrasound imaging device in one embodiment of the present disclosure.

FIG. 13 shows a structural block diagram of a computer device in one embodiment of the present disclosure.

FIG. 14 shows a schematic structural diagram of an embodiment of a side-looking 4D intracardiac echocardiography imaging system in one embodiment of the present disclosure.

FIG. 15 shows a schematic structural diagram of an embodiment of a forward-looking 4D intracardiac echocardiography imaging system in one embodiment of the present disclosure.

FIG. 16 shows a schematic diagram of different ultrasound transducers in one embodiment of the present disclosure.

FIG. 17 shows a schematic diagram of a 2D scanning and imaging method without rotation in one embodiment.

FIG. 18 shows a schematic diagram of a 3D scanning and imaging during rotational imaging of a forward-looking 4D intracardiac echocardiography imaging system in one embodiment of the present disclosure.

## DETAILED DESCRIPTION OF THE EMBODIMENTS

The following describes the technical solutions in the embodiments of the present disclosure with reference to the accompanying drawings. Apparently, the described embodiments are some of the embodiments of the present disclosure rather than all the embodiments. All other embodiments obtained by a person of ordinary skill in the art based on the embodiments of the present disclosure without creative efforts shall fall within the protection scope of the present disclosure.

It should be noted that all directional indications (such as up, down, left, right, front, back) involved in the embodiments of the present disclosure are only used to explain the relative positional, motion states, and the like between various components in specific postures (as shown in the drawings). If the specific postures change, the directional indications also change correspondingly.

In addition, the descriptions of the terms "first", "second", and the like in the present disclosure are used for descriptive purposes only and are not to be construed as indicating or implying relative importance or to implicitly indicate the number of technical features indicated. From this, features defined as "first" and "second" may explicitly or implicitly include at least one feature. In addition, the technical solutions between the various embodiments can be combined with each other, but need to be implemented by those of ordinary skill in the art.

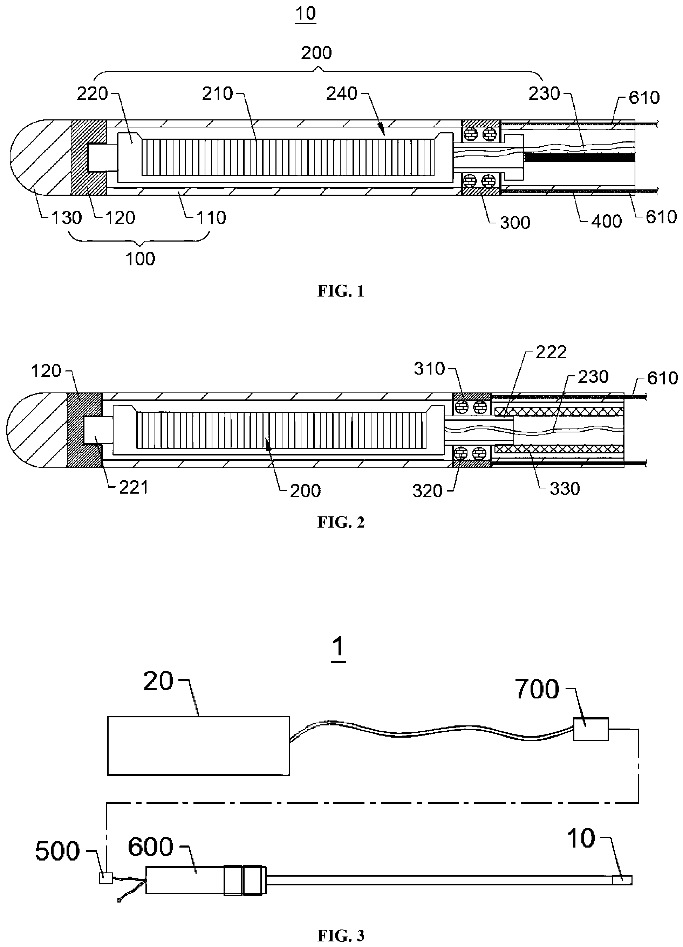

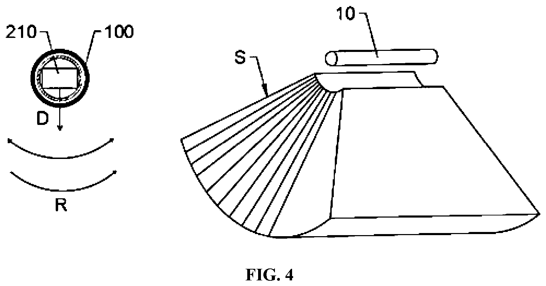

As shown in FIG. 1 to FIG. 4, an embodiment of the present disclosure provides a 4D ICE catheter 10, including a sheath 100, an ultrasound transducer assembly 200, a bearing 300, and an external lumen 400. An internal lumen is provided inside the sheath 100. The ultrasound transducer assembly 200 is rotatably connected to the sheath 100 and is suspended in the internal lumen. The bearing 300 is connected to the ultrasound transducer assembly 200 and an external steering handle. The external lumen 400 is connected to the bearing 300 and is located on one side of the bearing 300 that is away from the ultrasound transducer assembly 200.

In the 4D ICE catheter 10 of this embodiment, the ultrasound transducer assembly 200 is configured to rotate with the sheath 100, and the ultrasound transducer assembly 200 can be driven to rotate relative to the static sheath 100 through the bearing 300, to achieve 3D scanning detection.

Specifically, the ultrasound transducer assembly 200 includes an ultrasound transducer 210, a mounting rack 220, and a signal cable 230. The mounting rack 220 is rotatably connected to the sheath 100. The ultrasound transducer 210 is arranged on the mounting rack 220. The signal cable 230 is electrically connected to the ultrasound transducer 210 and is configured to be connected to an external ultrasound console 20.

In this embodiment, a mounting rack 220 is suspended in the internal lumen of the sheath 100. The mounting rack 220 can be connected to and driven to rotate by a bearing 300. In this case, the mounting rack 220 can drive the ultrasound transducer 210 to rotate relative to the static sheath 100. By rotating the ultrasound transducer 210, the ultrasound transducer 210 can be driven to swing in an R direction, so that the ultrasound transducer 210 can perform detection and identification within an S imaging space. The signal cable 230 can be configured to transmit a corresponding signal in the ultrasound transducer 210. The signal includes, but not limited to, a control signal of the ultrasound transducer 210, a analog signal received by the ultrasound transducer 210, and the like.

Further, the sheath 100 further internally includes a coupling medium 240 which fills the internal lumen and wraps around the ultrasound transducer assembly 200. The coupling medium 240 is usually liquid.

In this embodiment, the coupling medium 240 can be, but is not limited to, water, silicone oil, glycerin, or the like. By injecting the coupling medium 240 inside the sheath 100, ultrasound energy emitted by the ultrasound transducer 210 can be conducted through the coupling medium 240 to minimize the acoustic energy loss during transmission.

It can be understood that the coupling medium 240 fills the internal lumen around the ultrasound transducer assembly 200.

Specifically, the sheath 100 includes an acoustic housing 110 and a supporting component 120. The supporting component 120 is connected to the acoustic housing 110 and encloses the internal lumen together with the acoustic housing 110. One end of the mounting rack 220 is rotatably connected to the supporting component 120. The bearing 300 includes a bearing body 310 and a sealing ring 320. The sealing ring 320 is located on an inner side of the bearing body 310. The bearing body 310 is connected to the mounting rack 220 and is connected to the acoustic housing 110 and the steering handle 600. The steering handle 600 is configured to deflect the acoustic housing 110. The other end of the mounting rack 220 is arranged to pass through the sealing ring 320 and connected to a drive unit 700 on the steering handle 600.

Thus, the acoustic housing 110 can be used as part of the sheath 100 and contains an internal lumen. In this case, the supporting component 120 is connected to the end of the acoustic housing 110 and is used as a connecting carrier for the mounting rack 220. In this case, the mounting rack 220 only rotates relative to the supporting component 120, thereby minimizing the friction resistance due to contact between the ultrasound transducer assembly 200 and the acoustic housing 110. During the rotation of the mounting rack 220 relative to the sheath 100, the sealing ring 320 can be configured to support one end of the mounting rack 220 away from the supporting component 120. In this case, two ends of the mounting rack 220 can be supported by the supporting component 120 and the sealing ring 320 respectively, so that the mounting rack 220 can rotate stably relative to the sheath 100 to avoid wobbling to minimize the imaging artifacts.

In one embodiment, the mounting rack 220 includes a first rotating shaft 221 and a second rotating shaft 222 at two opposite ends of the mounting rack 220. The first rotating shaft 221 is connected to the supporting component 120. The second rotating shaft 222 extends to the sealing ring 320 and is connected to the drive unit 700.

In this embodiment, the first rotating shaft 221 protrudes from an end portion of the mounting rack 220. The second rotating shaft 222 extends outward from another end of the mounting rack 220. The first rotating shaft 221 can be inserted into the supporting component 120, and radial and axial movements of the first rotating shaft 221 are positioned through the supporting component 120. Furthermore, the mounting rack 220 can fit into the first rotating shaft 221 during rotation for supporting. In this case, the second rotating shaft 222 is connected to the sealing ring 320 and is supported by the sealing ring 320. Therefore, the mounting rack 220 is supported by the first rotating shaft 221 and the second rotating shaft 222 respectively, so that the entire mounting rack 220 can be suspended within the acoustic housing 110.

Further, the bearing 300 further includes a torque coil 330 which is respectively connected to the mounting rack 220 and the drive unit 700.

Thus, the sealing ring 320 can be sealed at one end of the acoustic housing 110 from the supporting component 120. In this case, two opposite ends of the internal lumen are respectively sealed by the supporting component 120 and the sealing ring 320, to minimize the leakage of the coupling medium 240.

Referring to FIG. 2, in another implementation, the torque coil 330 is of a hollow structure. The signal cable 230 passes through the torque coil 330 and is connected to the ultrasound connector 500. The ultrasound connector 500 is connected to the ultrasound console 20.

In this embodiment, by setting the torque coil 330 as the hollow structure, the signal cable 230 can pass through the torque coil 330 and is connected to the external ultrasound console 20 through the connector 500. In this case, the torque coil 330 can also protect the signal cable 230.

Referring to FIG. 2, in one embodiment, the torque coil 330 is of a solid structure, and the signal cable 230 is parallel to the torque coil 330.

In this embodiment, the torque coil 330 can be configured only to drive the mounting rack 220 to rotate, and in this case, the signal cable 230 can be parallel to the torque coil 330, or can be arranged around the torque coil 330.

In one embodiment, the 4D ICE catheter 10 further includes the steering handle 600 and the drive unit 700. The steering handle 600 includes a pull wire 610. The pull wire 610 is embedded within the wall of the sheath 100 and is individually connected to the steering handle 600 and the bearing 300. The steering handle 600 is configured to pull the pull wire 61, thereby deflecting the sheath 100. The sheath 100 is flexible and is soft in the distal end, so that the distal end of the sheath 100 deflects. The drive unit 700 is connected to the ultrasound transducer assembly 200 and is configured to drive the ultrasound transducer assembly 200 to uniformly rotate within the sheath 100 uni-directionally or bi-directionally.

Thus, by using the steering handle 600 to pull the pull wire 610, the sheath 300 can be deflected for switching the view angle. The drive unit 700 can drive the mounting rack 220 to rotate relative to the sheath 100 through the torque coil 330, thereby driving the ultrasound transducer 210 to rotate.

Further, the sheath 100 further includes a catheter tip 130. The catheter tip 130 is connected to the distal end of the supporting component 120 away from the acoustic housing 110, and a front end of the catheter tip 130 is of a curved surface structure.

In this arrangement, an end portion of the sheath 100 can be a cambered surface. When the 4D ICE catheter 10 is placed into a blood vessel or a human body, the friction between the sheath 100 and human tissues can be reduced, and the forward-movement resistance of the sheath 100 can be alleviated, thus avoiding injury caused by the sheath 100 to the human tissues during delivery.

The present disclosure further provides an ultrasound imaging equipment 1, including an ultrasound console 20 and the 4D ICE catheter 10 according to any one of the above embodiments. The 4D ICE catheter 10 further includes an ultrasound connector 500, which is electrically connected to the drive unit 700. The steering handle 600 is connected to the sheath 100 and the bearing 300.

It can be understood that in the ultrasound imaging equipment 1 of this embodiment, the 4D ICE catheter 10 of this embodiment achieves 3D scanning by rotating the ultrasound transducer assembly 200 within the sheath 100 by the bearing 300.

In the embodiments of the present disclosure, the ultrasound imaging equipment has two imaging modes: a 2D imaging mode and a 4D imaging mode. When the bearing 300 rotates slowly or remains stationary, the ultrasound imaging equipment is in 2D imaging mode. When the bearing 300 rotates rapidly, the ultrasound imaging equipment is in the 4D imaging mode.

A position sensor is integrated in the transducer of the ultrasound transducer assembly. When the bearing rotates slowly, the ultrasound imaging equipment can by synchronized by an electrocardiogram gating signal to perform image segmentation and 3D surface reconstruction on a series of 2D ultrasound images captured at different rotational positions at the cardiac end-diastolic phase, thus obtaining a 3D model of each specific cardiac structure (such as the pulmonary vein, the left atrium, the left ventricle, the right atrium, and the right ventricle) at the cardiac end-diastolic phase.

The image segmentation employs an AI-based deep learning neural network method or can employ a traditional image segmentation method, such as the SNAKES algorithm.

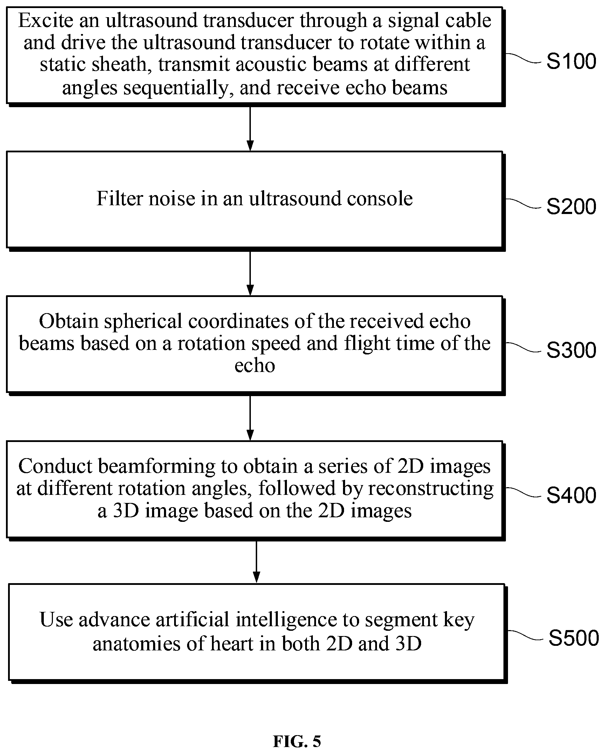

The present disclosure further provides an ultrasound imaging method, which uses the 4D ICE catheter 10 in any one of the above embodiments or the ultrasound imaging equipment 1 in any one of the above embodiments. Referring to FIG. 5, the ultrasound imaging method includes the following steps:

Step S100: Exciting the ultrasound transducer 210 through a signal cable 230 and driving it to rotate within the static sheath 100, transmitting acoustic beam along different angles sequentially and receiving echo beams.

Step S200: Filter noise in the ultrasound console 20.

Step S300: Obtaining the spherical coordinates of received echo beams based on the rotation speed and flight time of the echo.

Step S400: Conducting beamforming to obtain a series of 2D images at different rotation angles, followed by reconstructing a 3D image based on the 2D images.

Step S500: Advance artificial intelligence is used to segment key anatomies of heart in both 2D and 3D.

In the ultrasound imaging method of this embodiment, the ultrasound transducer 210 can perform scanning uni-directionally or bi-directionally. In this case, the ultrasound transducer 210 can obtain a series of 2D images, followed by three-dimensional reconstruction, denoising and rendering.

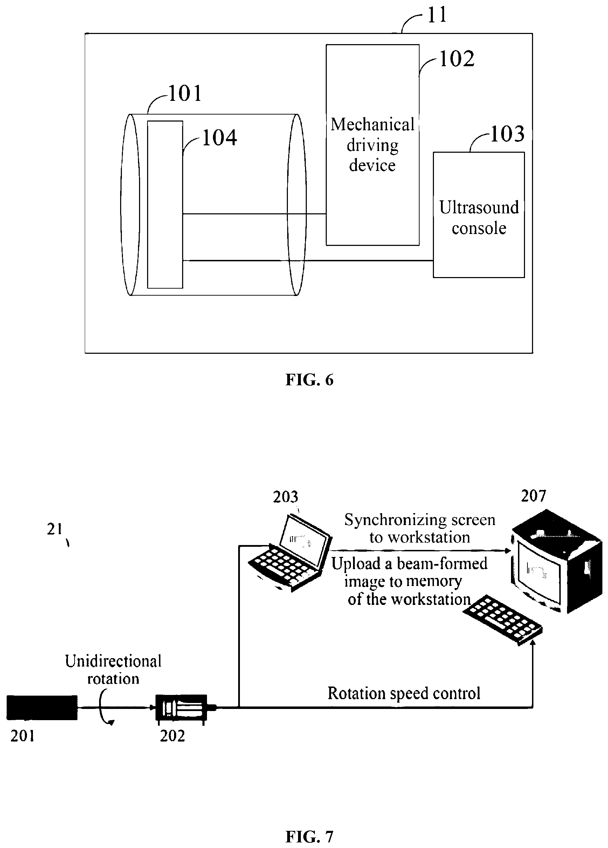

Referring to FIG. 6 to FIG. 10, an embodiment of the present disclosure further provides a 4D ICE imaging system, which refers to 4D imaging.

As shown in FIG. 6, the 4D ICE imaging system 11 at least includes: an interventional catheter 101, a mechanical driving device 102 including a motor, and an ultrasound console 103. The interventional catheter 101 includes a cavity located at the distal end and a miniature ultrasound transducer 104 located in the cavity. The mechanical driving device 102 is at a proximal end of the interventional catheter 101; The mechanical driving device 102 and the ultrasound console 103 are connected to the miniature ultrasound transducer 104, respectively.

It should be noted that the miniature ultrasound transducer 104 at a distal end of the interventional catheter 101 will enter the heart for 4D cardiac ultrasound imaging. The interventional catheter 101 can be inserted through peripheral vein, or other peripheral vessels. The imaging target includes, but is not limited to, the cardiac chamber, large vessels and other cardiovascular interventional catheter. The steering handle remain outside of the body during intervention. The end that is inserted into the body is referred to as a distal end, and the end that is reserved outside the body is referred to as a proximal end, namely the distal end and proximal end of the interventional catheter 101 in this embodiment. The interventional catheter 101 is referred to relative to a user operating the interventional catheter, such as the physician.

Further, the ultrasound console 103 is configured to: activate the transducer 104 to emit the acoustic beam, followed by receiving the reflected echoes and image postprocessing.

That is, the miniature ultrasound transducer 104 is configured to intermittently emit the acoustic wave driving signal outputted by the ultrasound console 103 for 2D imaging. Meanwhile, the mechanical driving device 102 is configured to rotate, when the miniature ultrasound transducer 104 emits the acoustic wave driving signal activated by the ultrasound console 103, the miniature ultrasound transducer 104 to rotate uniformly within the interventional catheter 101. Thus, the miniature ultrasound transducer 104 is further configured to: receive the activation signal outputted by the ultrasound console 103, and transmit the reflected echo signal to the ultrasound console 103. The ultrasound console 103 is further configured to perform signal post-processing and image processing on the received echo signal to generate 4D ultrasound imaging, rendering, and dynamic displaying.

The rotation of the mechanical driving device 102 can change an scanning angle of the acoustic beam around the elevation direction. The system works at 2D mode when the transducer is static or slowly rotating, while at 4D mode when the mechanical driving device 102 rotates fast, usually at a range between 10 rps and 50rps.

Existing 4D intracardiac echocardiography (ICE) typically employs a matrix array transducer which includes numerous ultrasound transducer elements arranged in two-direction for electronically deflecting ultrasound beams. The matrix array transducer can achieve real-time three-dimensional imaging at high fabrication cost. In another aspect, the transducer includes a large number of ultrasound elements. Each array element requires a separate lead wire. In order to reduce the number of wire connections, it is usually necessary to integrate a single-use application specific integrated circuit (ASIC) with the matrix array transducer. The ASIC development and production costs are high, with the risk of power dissipation and heating which is challenging for biomedical applications.

Therefore, the current implementation of 4D imaging has the problems such as high costs, large equipment volume, large amount of processed data, and high computation complexity.

The 4D intracardiac echocardiography imaging system of this embodiment can achieve4D ultrasound imaging using a simple transducer and cable connection method, thus is much cost efficient than the matrix array transducer 4D imaging method.

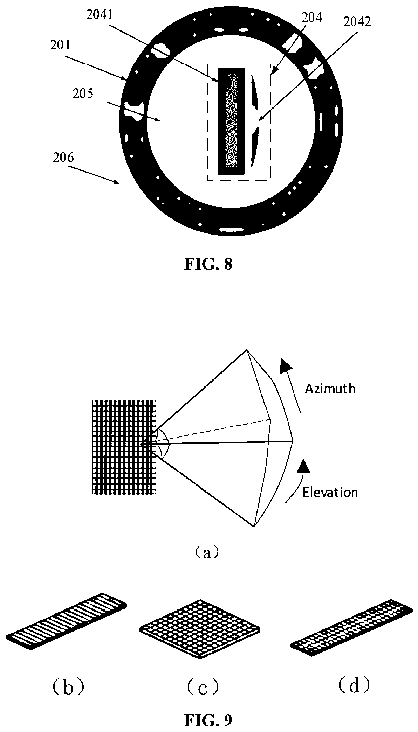

Referring to FIG. 7 and FIG. 8, FIG. 7 is another schematic structural diagram of a 4D intracardiac echocardiography imaging system in an embodiment of the present disclosure, and FIG. 8 is a schematic structural diagram of an ultrasound transducer in an embodiment of the present disclosure. FIG. 8 can also be regarded as a cross-sectional view of an interventional catheter. Further, as shown in FIG. 7 and FIG. 8, the 4D intracardiac echocardiography imaging system 21 at least includes: an interventional catheter 201, a mechanical driving device 202, and an ultrasound console 203. The interventional catheter 201 includes a cavity located at the distal end with a miniature ultrasound transducer 204 located in the cavity. The mechanical driving device 202 set at a proximal end of the interventional catheter 201; The mechanical driving device 202 and the ultrasound console 203 are connected to the miniature ultrasound transducer 204.

The ultrasound console 203 is configured to output an activation signal. The miniature ultrasound transducer 204 is configured to intermittently emit the acoustic beam. The mechanical driving device 202 is configured to rotate, when the miniature ultrasound transducer 204 emits the acoustic beam, the miniature ultrasound transducer 204 uniformly within the interventional catheter 201, so as to perform 4D scanning and imaging on different rotation angles of the imaging target. The miniature ultrasound transducer 204 is further configured to: receive the reflected echo signal, and transmit the received echo signal to the ultrasound console 203. The ultrasound console 203 is further configured to perform signal post-processing and image processing on the received echo signal to generate the 4D ultrasound cardiac image. For example, a 4D image is obtained based on the echo signal, and the generated image is displayed on a monitor after denoising and rendering.

It should be noted that content of the interventional catheter 201, the mechanical driving device 202, the ultrasound console 203, and the miniature ultrasound transducer 204 are similar to content to the interventional catheter 101, the mechanical driving device 102, the ultrasound console 103, and the miniature ultrasound transducer 104 shown in FIG. 6. To avoid repetition, no elaboration will be made here. For details, refer to the content of the interventional catheter 101, the mechanical driving device 102, the ultrasound console 103, and the miniature ultrasound transducer 104 shown in FIG. 6.

Further, the miniature ultrasound transducer 204 includes a transducer 2041 and an acoustic lens 2042. The acoustic lens 2042 covers on the transducer 2041. The acoustic lens 2042 focuses ultrasonic waves emitted by the transducer 2041 to enhance a signal-to-noise ratio of the ultrasonic waves at a certain deep focusing position. Acoustic focusing in a direction perpendicular to an imaging plane is adjusted through the acoustic lens 2042 on the transducer. An imaging window with the maximum rotation direction of 360° can be achieved by adjusting the imaging window in a rotation direction through software. The miniature ultrasound transducer 204 is a phased array transducer for 2D imaging, or a plurality of phased array transducers for 2D imaging, which are combined in a preset rotation angle. A combination manner can be back-to-back arrangement. For example, referring to FIG. 9, FIG. 9 is a schematic diagram of imaging of a matrix array transducer in an embodiment of the present disclosure, where FIG. 9(a) shows volumetric imaging of a matrix ultrasound transducer; FIG. 9(b) shows a linear-phase array transducer; FIG. 9(c) shows a 2D matrix array; and FIG. 9(d) shows a 1.5D matrix array.

It should be noted that a commonly used 4D ICE transducer usually employs area array ultrasound transducers such as the 2D area array transducer and the 1.5D transducer in FIG. 9(c) and FIG. 9(d). These transducers are arranged two-dimensionally and can perform dynamic 3D volume imaging.

Moreover, the above matrix array transducer has numerous elements, usually 1024 or 840 array elements. The smaller the ICE transducer, the less the puncture injury to the patient during intervention. About 1000 array elements are integrated on the transducer with a width of only about 3 mm. The integration degree and the technology of the transducer are complicated and the cost is high. In another aspect, each array element requires a separate lead wire to achieve separate excitation and signal receiving, and about 1000 lead wires cannot be led out of a catheter of about 3 mm. In order to reduce the number of the lead wires, an ASIC needs to be added on the transducer for signal preprocessing, thus outputting a preprocessed signal from the catheter with fewer lead wires. These processes lead to high difficulty and high costs of array 4D ICE . Meanwhile, due to a large amount of data of the transducer, complex post-processing, and difficulty in solving the heating problem of the ASIC, the costs of the console is high.

The present disclosure provides a technology for achieving 4D imaging of the heart through mechanical rotation in order to reduce the costs of 4D ICE of the matrix array. An ultrasound transducer used is the 2D imaging transducer (linear-phase array transducer) shown in FIG. 9(b). The transducer includes a small number of transducer units arranged linearly. The deflection, scanning, and imaging in the direction perpendicular to the imaging plane are achieved through mechanical rotation, thus greatly lowering the difficulty of integration and reducing the number of transducers. In another aspect, due to the large amount of data and high computation complexity, the present disclosure provides a three-dimensional image reconstruction algorithm based on high-performance parallel computation, which can significantly improve the post-processing speed of mechanical 4D ICE. It can be understood that the ultrasound transducer in this embodiment can be a 2D imaging transducer or a 2D matrix array ultrasound transducer or another transducer such as an ultrasound linear array , circular arrays, convex arrays and 1.5D transducer. The transducer material is PZT(porous lead zirconate titanate) ceramic, or a composite material, a PMUT(piezoelectric micromachined ultrasound transducer) material, CMUT(capacitive micromachined ultrasound transducer) material, or another novel transducer material.

Further, the cavity of the interventional catheter 201 is filled with normal saline or another coupling liquid (such as glycerol) 205. A filling manner can be to fill the cavity of the interventional catheter with the normal saline through a syringe at the proximal end of the interventional catheter, to achieve acoustic coupling between the transducer and an external lumen. For example, the interventional catheter 201 can be a Pebax catheter. After the interventional catheter is inserted into the body, an outer wall is generally in contact with body fluid 206. The body fluid 206 can be blood. The transducer and the acoustic lens rotate unidirectionally in the Pebax catheter, while the catheter remains stationary.

For example, the above rotation can be uni-directional rotation or bi-directional rotation.

In one embodiment, an inner wall of the interventional catheter 201 includes at least four pull wires with an interval of 90° (not shown), and a handle (not shown) is also disposed at the proximal end of the interventional catheter. The handle is configured to pull at least one pull wire to adjust the tightness of the pull wire, thus achieving angle deflection of the miniature ultrasound transducer, namely, bending in four directions, to image different positions of an imaging target. The imaging target includes, but is not limited to, heart, cardiac chamber, and the like. A knob may be disposed on the handle. The tightness of the pull wire is adjusted by the knob. Since a front end of the catheter is made of a flexible material, and a rear end is made of a relatively hard material , the pull wire is embedded in the catheter. By pulling the pull wire, the flexible material of the front end bends, while the relatively hard material of the rear end does not bend, thus achieving deflection. For example, a steering handle may be a four-way or two-way bendable handle.

In one embodiment, the mechanical driving device 202 includes a motor, a multi-channel slip ring, and a console end connector; the motor is fixedly connected to a stator of the multi-channel slip ring; the console end connector is fixedly connected to a rotor of the multi-channel slip ring; the console end connector is connected to a catheter-side connector; the motor drives the rotor of the multi-channel slip ring to rotate through mechanical transmission; and the motor, the multi-channel slip ring, and the console end connector are all reusable.

It should be noted that since the motor and the multi-channel slip ring of the mechanical driving device will not enter the body, the mechanical driving device can be reused for multiple times. Moreover, the motor and the multi-channel slip ring are expensive components. This can also save the costs and achieve rational resource utilization.

Further, the motor of this solution is disposed on the ultrasound console for repeated use. The linear-phase array ultrasound transducer located at a tip end of the catheter is driven to rotate by a torque coil located within the catheter, thereby achieving low-cost 4D imaging. In order to reduce the performance requirement and the costs of the ultrasound console, and improve the speed of post-processing of mechanical 4D imaging, this patent provides a 3D reconstruction algorithm based on high-performance parallel computation. Algorithms that require high computing power, such as 3D reconstruction and rendering, are completed on a back-end parallel computation workstation (such as CPU (central processing unit) workstation or GPU(graphics processing unit)). Therefore, a low-cost portable ultrasound console can be used. This reduces the equipment costs of hospitals, which will be discussed later.

In one embodiment, the 4D intracardiac echocardiography imaging system further includes a torque coil and a catheter-side connector. The torque coil is disposed in the cavity of the interventional catheter and is fixedly connected to the miniature ultrasound transducer. Furthermore, the torque coil is fixedly connected to the catheter-side connector, and the rotation of the catheter-side connector drives the torque coil and the transducer to rotate. The miniature ultrasound transducer, the torque coil, and catheter-side connector are all disposable.

It should be noted that the miniature ultrasound transducer, the torque coil, and the catheter-side connector for human intervention can be replaced. For example, the miniature ultrasound transducer, the torque coil, and the catheter-side connector will be replaced for different participants, so they are disposable. Uniform unidirectional rotation of the transducer within the catheter is achieved through the slip ring and the torque coil, to avoid twisting of a lead wire of the transducer caused by the unidirectional rotation. The motor is placed outside a proximal catheter and reused. This reduces the costs of consumables and the size of the catheter. Furthermore, a large-sized closed-loop control motor can be used to improve the accuracy of rotational imaging.

The fixed connection includes but is not limited to welding. As there is no relative motion between the torque coil and the transducer, welding is generally employed. The connectors include a catheter end connector and a console end connector. Usually, the spring and the catheter end connector are also welded.

Further, in order to improve imaging efficiency, the 4D intracardiac echocardiography imaging system may further include a workstation 207 for 4D ultrasound imaging. The ultrasound console can synchronize a screen to the workstation, so that a beam-formed image is transmitted to a memory of the workstation and a subsequent ultrasound imaging method is performed to obtain a 4D ultrasound image of the imaging target. The ultrasound imaging method will be described later. In addition, a rotation speed of the motor can be controlled by the workstation, which is not limited here.

This embodiment of the present disclosure provides the 4D intracardiac echocardiography imaging system. The 4D intracardiac echocardiography imaging system at least includes: the interventional catheter, the mechanical driving device, and the ultrasound console. The distal end of the interventional catheter contains the miniature ultrasound transducer. The mechanical driving device includes the motor and the multi-channel slip ring and is disposed at the proximal end of the interventional catheter. The mechanical driving device and the ultrasound console are respectively connected to the miniature ultrasound transducer. The ultrasound console is configured to output an activation signal. The miniature ultrasound transducer is configured to emit the acoustic beam intermittently. The mechanical driving device is configured to rotate, when the miniature ultrasound transducer emits the acoustic beam, the miniature ultrasound transducer to rotate uniformly within the interventional catheter, so as to perform mechanical 4D scanning and imaging on different rotation angles of the imaging target. The miniature ultrasound transducer is further configured to: receive the reflected echo signal, and transmit the received echo signal to the ultrasound console. The ultrasound console is further configured to determine the 4D ultrasound image of the imaging target. The closed-loop control motor is located at the proximal end of the catheter and has an adjustable rotation speed. Through its unidirectional rotation, the motor drives the rotor portion of the slip ring to rotate, and through the connector, the torque coil and the transducer are driven to rotate uniformly and unidirectionally. The handle controls an imaging window angle of the transducer through the pull wire. The ultrasound transducer rapidly rotates uniformly and unidirectionally while performing rapid imaging. The collected signal is transmitted to a portable ultrasound console for beamforming, and the data after beamforming is transmitted to the workstation for interpolation 3D reconstruction, rendering, and displaying based on high-performance parallel computation.

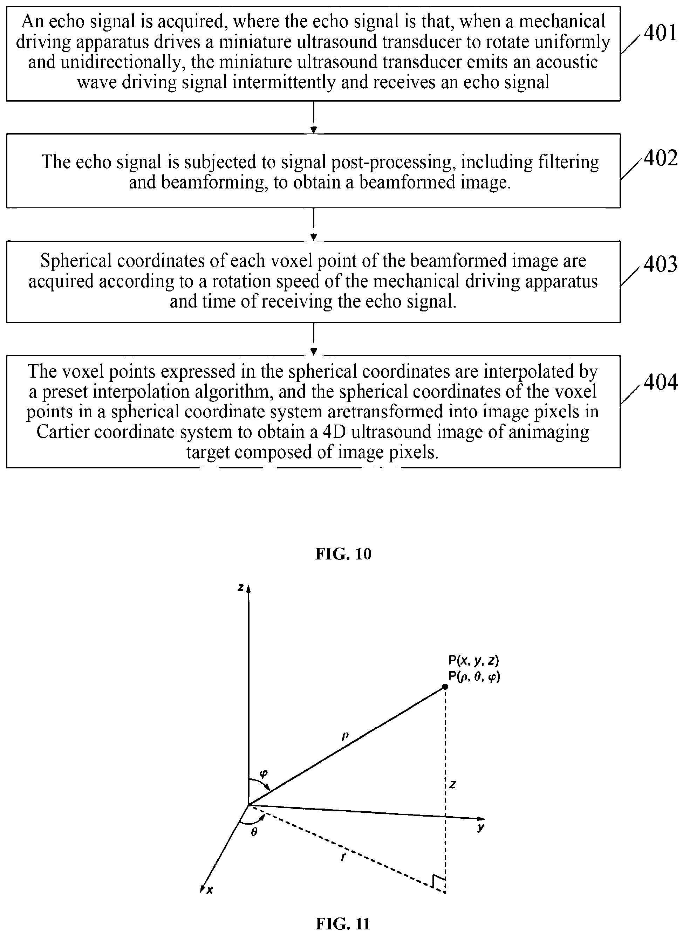

Referring to FIG. 10, FIG. 10 is a flowchart of an ultrasound imaging method in an embodiment of the present disclosure. As shown in FIG. 10, the ultrasound imaging method is applied to the above 4D intracardiac echocardiography imaging system. The ultrasound imaging method includes:

401:An echo signal is acquired, where the echo signal is that, when a mechanical driving device drives a miniature ultrasonic transducer to rotate uniformly and unidirectionally, the miniature ultrasonic transducer emits an acoustic wave driving signal intermittently and receives an echo signal.

It should be noted that an executive agent of the above ultrasound imaging method can be the ultrasound console or the workstation, which is not limited here. Since the ICE imaging system of the present disclosure is a multifunctional imaging system, it can be compatible with two imaging modes: 2D ICE and 4D ICE. In the 2D imaging mode, due to a small amount of data and computation, imaging can be directly completed on the portable ultrasound console, without the mechanical rotation of the motor, the slip ring, and the transducer. In the 4D imaging mode, the mechanical rotation is required, and the algorithm and the post-processing are complex. The following mainly explains the 4D imaging mode. This implementation is the 4D ultrasound imaging. Therefore, it is necessary to obtain the echo signal of the emitted and received acoustic wave driving signal for imaging. In addition, in order for ultrasound imaging, the echo signal is received after the miniature ultrasound transducer intermittently emitting the acoustic wave driving signal when the mechanical driving device drives the miniature ultrasound transducer to rotate uniformly and unidirectionally.

For example, the ultrasound transducer in this embodiment is a linear-phase array ultrasound transducer. The transducer is shown in FIG. 9(b). As the transducer can only achieve deflection and focusing within a 2D imaging plane, an acoustic lens is used on the ultrasound transducer and rotates in the catheter. Furthermore, in order to avoid a gap between the transducer and the catheter from being filled with air, which may affect the sound transmission, normal saline is injected into the catheter. Acoustic waves are refracted within the acoustic lens to achieve geometric focusing in a direction perpendicular to a vertical plane, thereby improving an imaging signal-to-noise ratio and contrast at the focused position.

In the present disclosure, the closed-loop control motor located at the proximal end of the catheter achieves high-precision unidirectional rotation, thus driving the multi-channel slip ring, the torque coil, and the transducer to rotate uniformly and unidirectionally within the catheter. While rotating unidirectionally, the ultrasound transducer performs high-speed imaging. In order to adjust an angle of an imaging field of view, a tip end of the catheter can be pulled to deflect through a steering handle and a pull wire in the catheter.

For example, after receiving a pressure signal, the ultrasound transducer converts the pressure signal into an analog electrical signal and transmits the analog electrical signal to the portable ultrasound console through a lead wire and the slip ring to obtain the acoustic wave signal. The ultrasound console performs beamforming. Generally, the ultrasound console is a field programmable gate array (FPGA), and some soft beams are formed on a central processing unit (CPU) or a graphics processing unit (GPU) of the ultrasound console, and beam-formed data (i.e., images of voxel points located on the beams), deflection angles of the beams, and a deflection angle of the transducer are transmitted to the workstation through a high-speed data interface. The high-speed data interface includes, but is not limited to, a universal serial bus (USB), a network cable, a peripheral component interconnect express (PCI-E), a thunderbolt, and the like. Assuming that a number of beams imaged per second is M and N beams are required for one instance of 3D image reconstruction, a volumetric imaging frame rate of 4D ICE is M/N.

402: The echo signal is subjected to signal post-processing, including filtering and beamforming, to obtain a beamformed image.

Further, the obtained echo signal needs to be subjected to filtering and beamforming. Specifically, the received echo signal is subjected to signal post-processing, including filtering and beamforming, to obtain a beamformed image. Signal post-processing includes, but is not limited to, filtering, analog-digital conversion, gain, amplification, beamforming, and other signal processing.

For example, the ultrasonic transducer converts a received pressure signal into an analog electrical signal. The analog electrical signal is transmitted to the portable ultrasonic console through a lead wire and a slip ring, thus obtaining an acoustic wave signal. The beamforming is performed on the ultrasonic console (in general, the ultrasonic console is FPGA, ultrasonic console for partial soft beamforming is on CPU or GPU), and the data after beamforming (i.e., the image of voxel points on the beam), a deflection angle of the beam and a deflection angle of the transducer are transmitted to the workstation through a high-speed data interface (e.g., USB, a network cable, PCI-E, and a thunderbolt). Assuming that the number of imaged beams per second is M and the construction of a three-dimensional image needs N beams, a volumetric Imaging frame rate of 4D ICE is M/N.

Cardiac imaging generally adopts focused ultrasound imaging, that is, each excitation and reception are in the form of linear acoustic waves, and multiple linear beams are emitted and received at different emission angles in turn to scan the whole imaging area, thus scanning and imaging the tissue. During 4D imaging, image data of multiple beams needs to be combined in the post processing to form a two-dimensional image, or a three-dimensional image (image reconstruction) which needs to be presented to doctors in real time for intraoperative guidance. Therefore, powerful computing power is required for post-processing three-dimensional reconstruction. The computing power of the traditional portable ultrasonic console is limited, while an expensive trolley ultrasonic console is usually used for the area matrix 4D ICE. In order to reduce the cost, the portable ultrasonic console can be used in the present disclosure, and the parallel computing-based image reconstruction algorithm can be performed to improve the post-processing speed.

403: Spherical coordinates of each voxel point of the beamformed image are acquired according to a rotation speed of the mechanical driving device and time of receiving the echo signal.

It should be noted that a voxel point P in the beam-formed image is represented as $(\rho, \theta, \varphi)$ in a spherical coordinate system, where $\rho$ represents a distance from the voxel point to a center of the transducer; $\theta$ represents a deflection angle of scanning of the imaging plane; and $\varphi$ represents the deflection angle of the mechanical rotation of the transducer. Therefore, for the beam-formed image, the spherical coordinates of the voxel points can be determined based on the rotation speed of the mechanical driving device and the time of receiving the echo signal.

404: The voxel points expressed in the spherical coordinates are interpolated by a preset interpolation algorithm, and the spherical coordinates of the voxel points in a spherical coordinate system are transformed into image pixels in Cartier coordinate system to obtain a 4D ultrasonic image of an imaging target composed of image pixels.

Further, the image pixels can be obtained by converting the spherical coordinates into Cartier coordinate system, thus achieving the reconstruction of the 4D ultrasonic imaging. Specifically, the voxel points expressed in the spherical coordinates are interpolated by a preset interpolation algorithm to determine the image pixels of the voxel points in the Cartier coordinate system, thus obtaining a 4D ultrasonic image of an imaging target composed of the image pixels.

It may be understood that the 4D ultrasonic images of the imaging target may also be displayed on the ultrasonic console or other display modules with display capability, thus providing surgical guidance for medical staff during interventional procedure and better navigate during surgery.

For example, referring to FIG. 11, FIG. 11 is a schematic diagram of a spherical coordinate system and a Cartier coordinate system in an embodiment of the present disclosure. A voxel point P is represented as $(\rho, \theta, \varphi)$ in the spherical coordinate system, where $\rho$ represents a distance from the voxel to a center of the transducer; $\theta$ represents a deflection angle of scanning of the imaging plane; and $\varphi$ represents the deflection angle of the mechanical rotation of the transducer. The point P is represented as (x, y, z) in the Cartier coordinate system.

It should be noted that in the 4D imaging mode of this embodiment, spherical coordinate data acquired by imaging of a plurality of ultrasound beams needs to be transmitted from the portable ultrasound console to the memory of the workstation, and is reconstructed into an image in the Cartier coordinate system through 3D interpolation. By convention, the array elements of the transducer are arranged along an x-axis and the transducer rotates around the x-axis, as shown in FIG. 10. This algorithm has the characteristics of a large volume of data and simple single-thread operation, making it suitable for parallel computation. The following is an interpolation 3D reconstruction algorithm based on high-performance parallel computation:

Assuming that the number of focused beams during imaging per second is M, an electronic deflection angle of adjacent beams in the imaging plane is d_$\theta$, and a mechanical deflection angle in the rotation direction is d_$\varphi$. Any position on any beam is represented as $(\rho_1, \theta_1, \varphi_1), \cdots, (\rho_M, \theta_M, \varphi_M)$ by using spherical coordinates, where $\rho$ cannot exceed a maximum imaging depth.

In a parallel computation architecture, each thread is used to calculate each voxel in the Cartier coordinate system. That is, each voxel on a 3D image is represented as a grid in the Cartier coordinate system, and its coordinates are represented as (x, y, z). The coordinates are converted to the spherical coordinate system according to the following formula:

$$\rho = \sqrt{x^2 + y^2 + z^2}$$

$$\theta = \text{atan}\left(\frac{y}{x}\right)$$

$$\phi = \text{asin}\left(\frac{\sqrt{x^2+y^2}}{\rho}\right)$$

Interpolation calculation is performed on each thread. For simplicity, the present disclosure uses trilinear interpolation calculation. That is, assuming $\rho_i < \rho \le \rho_{i+1}$, $\theta_j < \theta \le \theta_{j+1}$, $\varphi_k < \varphi \le \varphi_{k+1}$, the interpolation algorithm includes the following mathematical expression:

$C = V(\rho_i,\theta_j,\varphi_k)(1 - \rho_d)(1 - \theta_d)(1 - \varphi_d) + V(\rho_{i+1},\theta_j,\varphi_k)\rho_d(1 - \theta_d)(1 - \varphi_d) + V(\rho_i,\theta_j,\varphi_{k+1})(1 - \rho_d)(1 - \theta_d)(\varphi_d + V(\rho_{i+1},\theta_j,\varphi_{k+1})\rho_d(1 - \theta_d)\varphi_d + V(\rho_i,\theta_{j+1},\varphi_k)(1 - \rho_d)\theta_d(1 - \varphi_d) + V(\rho_{i+1},\theta_j + 1,\varphi_k)\rho_d\theta_d(1 - \varphi_d) + V(\rho_i,\theta_{j+1},\varphi_{k+1})(1 - \rho_d)\theta_d\varphi_d + V(\rho_{i+1},\theta_j + 1,\varphi_k)\rho_d\theta_d\varphi_d$:

Where $\rho_d = (\rho - \rho_i)/(\rho_{i+1} - \rho_i)$, $\theta_d = (\theta - \theta_j)/(\theta_{j+1} - \theta_j)$, $\varphi_d = (\varphi - \varphi_k)/(\varphi_{k+1} - \varphi_k)$, $(\rho_i,\theta_j,\varphi_k)$ represents the spherical coordinates, and C represents an image pixel in the Cartier coordinate system.

The above i+1, j+1, and k+1 are all coordinate points near i, j, k, for interpolation calculation on $(\rho_i,\theta_j,\varphi_k)$ to determine the image pixels of the points on the Cartier coordinate system (x, y, z).

Other interpolation algorithms, such as Kriging interpolation, polynomial interpolation, and spline function interpolation, can also be used for the above trilinear interpolation The above ultrasound imaging method can be implemented on a GPU workstation or a multi-core CPU or an FPGA, which can significantly improve the post-processing efficiency of the 3D interpolation reconstruction, achieve 4D cardiac imaging, and better implement surgical navigation.

The present disclosure provides the ultrasound imaging method applied to the above 4D ICE imaging system. The ultrasound imaging method includes: acquiring an echo signal, where the echo signal is that, when a mechanical driving device drives a miniature ultrasonic transducer to rotate uniformly and unidirectionally, the miniature ultrasonic transducer emits an acoustic wave driving signal intermittently and receives an echo signal; performing signal post-processing which includes filtering and beamforming on the echo signal to obtain a beamformed image; acquiring spherical coordinates of each voxel point of the beamformed image according to a rotation speed of the mechanical driving device and time of receiving the echo signal; and interpolating the voxel points expressed in the spherical coordinates by a preset interpolation algorithm, and transforming the spherical coordinates of the voxel points in a spherical coordinate system into image pixels in Cartier coordinate system to obtain a 4D ultrasonic image of an imaging target composed of the image pixels. Through above method, the ultrasonic imaging of an imaging target can be achieved, and through a three-dimensional reconstruction algorithm based on high-performance parallel computing, the three-dimensional reconstruction, rendering and other algorithms requiring high computing power can be completed on a back-end parallel computing workstation (such as GPU), so the portable ultrasonic console with low cost can be used and the device cost of the hospital can be reduced.

**In** summary, compared with the matrix array 4D ICE scheme, the mechanical 4D ICE solution provided in the present disclosure has the following advantages: 1. The cost of mechanical 4D ICE is about 10% of that of the matrix array 4D ICE scheme, and but the mechanical 4D ICE scheme has the same image quality as the matrix array 4D ICE scheme. 2. The mechanical 4D ICE scheme can achieve the maximum 360° imaging field of view in the rotation direction, and the imaging field of view is larger than that of the matrix array 4D ICE. 3. The mechanical 4D ICE scheme can use a portable ultrasonic console with low cost, and thus the admission cost is reduced. 4. The mechanical 4D ICE scheme, compared with an electronic 4D transducer, has less transducer heating, and is safer to a patient.

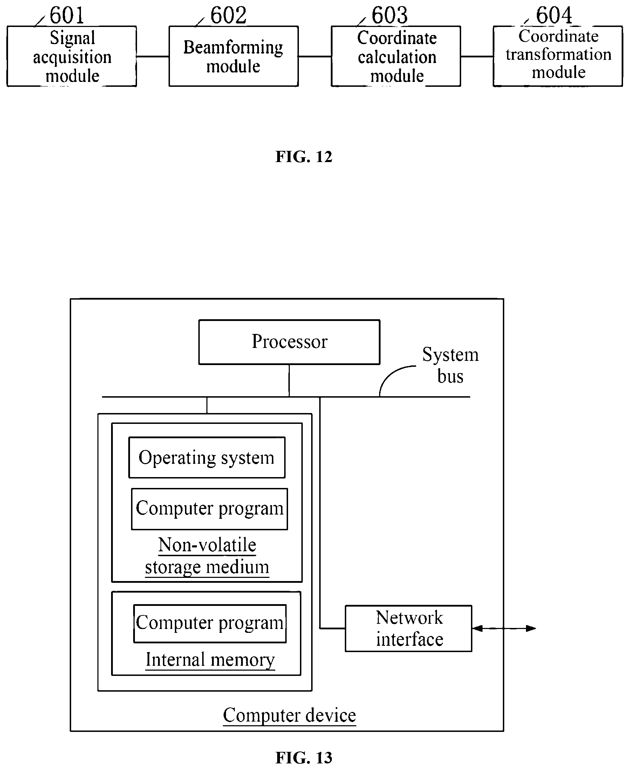

Referring to FIG. 12, FIG. 12 is structural block diagram of an ultrasound imaging device in an embodiment of the present disclosure. The ultrasound imaging device shown in FIG. 12 includes:

a signal acquisition module 601 configured to: obtain an echo signal, where the echo signal is received after the miniature ultrasound transducer intermittently emits a acoustic wave driving signal when the mechanical driving device drives the miniature ultrasound transducer to rotate uniformly and unidirectionally;

a beamforming module 602 configured to: filter the echo signal, and post-process a beam-formed signal to obtain a beam-formed image;

a coordinate calculation module 603 configured to: for the beam-formed image, obtain spherical coordinates of voxel points based on a rotation speed of the mechanical driving device and time of receiving the echo signal; and

**a** coordinate transformation module 604 configured to: perform, through a preset interpolation algorithm, interpolation processing on the voxel points represented in the spherical coordinates, convert the spherical coordinates of the voxel points in the spherical coordinate system into image pixels in a Cartier coordinate system, and obtain a 4D ultrasound image of an imaging target composed of the image pixels.

It should be noted that the device shown in FIG. 12 has similar content to the method shown in FIG. 10. To avoid repetition, no elaboration will be made here. For details, refer to the content of the method shown in FIG. 10 above.

Further, in order to ensure an image display effect, the above device may further include a rendering and display module configured to perform real-time displaying and 3D rendering on the obtained 4D ultrasound image on a display.

The present disclosure provides the ultrasound imaging device. The ultrasound imaging device includes: the signal acquisition module configured to obtain an echo signal, where the echo signal is that, when the mechanical driving device drives the miniature ultrasound transducer to rotate uniformly and unidirectionally, the miniature ultrasound transducer emits an acoustic wave driving signal intermittently and receives an echo signal; the beamforming module configured to perform signal post-processing which includes filtering and beamforming on the echo signal to obtain the beam-formed image; the coordinate calculation module configured to: obtain the spherical coordinates of the voxel points of the beam-formed image, based on the rotation speed of the mechanical driving device and the time of receiving the echo signal; and the coordinate transformation module configured to interpolate the voxel points represented in the spherical coordinates by a preset interpolation algorithm, and transform the spherical coordinates of the voxel points in the spherical coordinate system into the image pixels in the Cartier coordinate system to obtain a 4D ultrasound image of the imaging target composed of the image pixels. Through above device, the ultrasound imaging of the imaging target can be achieved, and through a 3D reconstruction algorithm based on high-performance parallel computing, the 3D reconstruction, rendering, and other algorithms requiring high computing power can be completed on a back-end paralled computing workstation (such as a GPU or a CPU). Therefore, a low-cost portable ultrasound console can be used and the device cost of the hospitals can be reduced..

FIG. 13 is a diagram of an internal structure of a computer device in an embodiment. The computer device may be specifically a terminal or a server. As shown in FIG. 13, the computer device includes a processor, a memory, and a network interface which are connected through a system bus. The memory includes a non-volatile storage medium and an internal memory. The non-volatile storage medium of the computer device stores an operating system and can also store a computer program. When the computer program is executed by the processor, the processor can be caused to implement the above method. The internal memory can also store a computer program, which, when executed by the processor, can cause the processor to perform the above method. A person skilled in the art can understand that the structure shown in FIG. 13 is merely a block diagram of a partial structure related to a solution in this application, and does not constitute a limitation on the computer device to which the solution in this application is applied. Specifically, the computer device may include more or fewer components than those shown in the figure, or some components may be combined, or a different component layout may be used.

In one embodiment, a computer device is provided, including a memory and a processor. The memory stores a computer program, and the computer program, when executed by the processor, enables the processor to execute the method shown in FIG. 5.

In one embodiment, a computer-readable storage medium is provided, having a computer program stored therein. The computer program, when executed by a processor, enables the processor to execute the method shown in FIG. 5.

Those skilled in the art can understand that all or part of the procedures of the methods of the foregoing embodiments may be implemented by a computer program instructing relevant hardware. The program may be stored in a non-volatile computer-readable storage medium. When the program is executed, the procedures of the foregoing method embodiments may be implemented. Any reference to the memory, the database, or other media used in the embodiments provided in the present application can include a non-volatile memory and/or a volatile memory. The non-volatile memory may include a read-only memory (ROM), a programmable ROM (PROM), an electrically programmable ROM (EPROM), an electrically erasable programmable ROM (EEPROM), or a flash memory. The volatile memory can include a random access memory (RAM) or an external cache memory. As an illustration rather than a limitation, the RAM can be obtained in various forms, such as a static RAM (SRAM), a dynamic RAM (DRAM), a synchronous DRAM (SDRAM), a double-data-rate SDRAM (DDRSDRAM), an enhanced SDRAM (ESDRAM), a synchronous link (Synchlink) DRAM (SLDRAM), a Rambus direct RAM (RDRAM), a direct RDRAM (DRDRAM), and a Rambus dynamic RAM (RDRAM).

An embodiment of the present disclosure further provides ultrasound imaging equipment which is a 4D intracardiac echocardiography imaging system. 4D cardiac imaging mentioned in this embodiment refers to dynamic 3D imaging and 4D imaging.

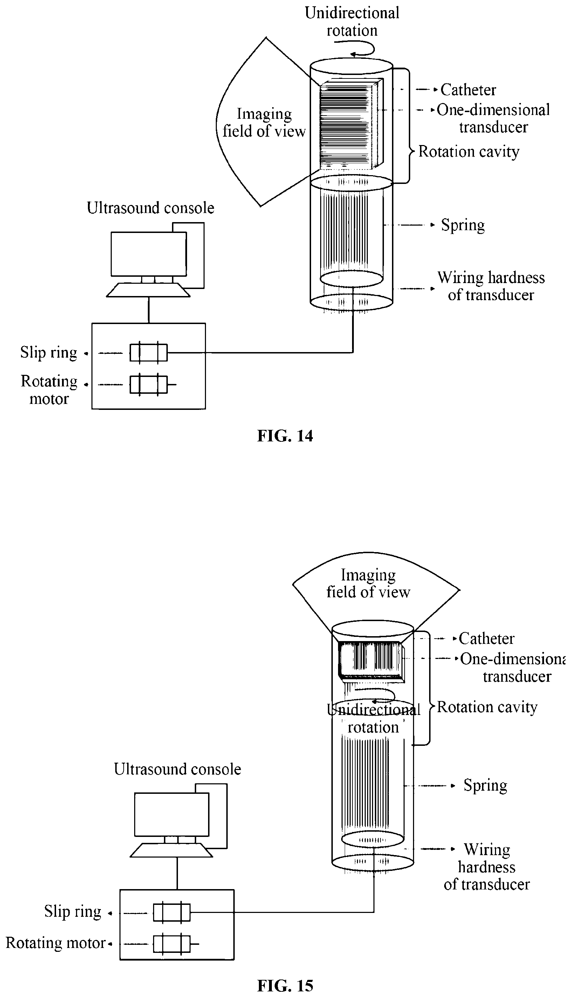

Referring to FIG. 14 to FIG. 18, the 4D intracardiac echocardiography imaging system at least includes: an ultrasound console, a slip ring, a console end connector, a catheter end connector, a motor, a transducer, a sheath, and a torque coil. The ultrasound console is connected to the console end connector through the slip ring, and the catheter end connector is connected to the transducer through the cable and the torque coil. The motor is configured to drive a rotor side of the slip ring to drive the connector to rotate at a high speed uniformly. The slip ring includes the rotor side and a stator side. The stator side is directly connected to the console end connector, and the rotor side is connected to the catheter. The motor drives the rotor side of the slip ring to rotate, thereby driving a rotor-side connector, the torque coil, and the transducer to rotate around a center of the transducer in the sheath. The torque coil is configured to drive the ultrasound transducer to rotate in the sheath, while the sheath remains stationary. The ultrasound transducer is configured to transmit ultrasound beams in different directions and receive the reflected echos. The transmitted beam can be focused beam or non focused beams including plane wave or divergent wave. The ultrasound console is configured to generate 2D images rotating around a central axis at a high speed with the motor and transducer rotating, and the 2D images formed at different rotation angles are 3D reconstructed followed by rendering and real-time display on monitors.

It can be understood that transducers used for four-dimensional intracardiac echocardiography imaging in clinical practice are all integrated with 2D matrix ultrasound sensors and ASICs. Intracardiac echocardiography catheters have high costs because they are disposable consumables.

This embodiment adopts a novel side-looking or forward-looking mechanical rotation 4D ICE imaging technology. A one-dimensional phased array ultrasound transducer is used. The motor is placed at on the console and drives the transducer to rotate through the torque coil to achieve 4D scanning, so that the costs of consumables are lower. The embodiments of the present disclosure adopt the forward-looking or side-looking imaging. Specifically, when the forward-looking imaging is used, the motor and the slip ring form two frames of four-dimensional images per rotation, with a higher imaging speed and a lower requirement on the rotation speeds of the motor and the slip ring (if a clinical requirement on a four-dimensional imaging frame rate is 20 vol/s, the rotation speeds of the motor and the slip ring only need to reach 10 rps).

In one embodiment, the slip ring includes a stator side and a rotor side. The rotor side is connected to the motor and rotates. The stator side is connected to the ultrasound console and is stationary. The rotor side and the stator side have relative motion and are electrically connected to each other.

In one embodiment, the transducer is a one-dimensional phased array transducer and rotates unidirectionally and uniformly at a high speed around a rotation center.

In one embodiment, the transducer at least includes configuration parameters in a scheme including center frequency, number of array elements, bandwidth, pitch and element arrangement.

Specifically, various parameters of a transducer array are not limited, and arrays in different schemes including center frequency, bandwidth, numbers of elements, pitch and element arrangements can be adjusted according to various application.

In one embodiment, in the side-looking imaging scheme, for every 360° rotation of the transducer around its center, a 3D image can be obtained. When the ultrasound four-dimensional imaging volumetric frame rate is set to f, the rotation speeds of the motor and the slip ring are f. In the forward-looking imaging scheme, for every 180° rotation of the transducer around its center, a 3D image can be obtained. When the ultrasound four-dimensional imaging volumetric frame rate is set to f, the rotation speeds of the motor and the slip ring are 0.5*f.

In one embodiment, the ultrasound console transmits ultrasound beams in different directions and receives the reflected echoes to form a 2D image, multiple 2D images are acquired at different rotation angle around the rotation center, followed by 3D reconstruction. Reconstructed four-dimensional image are rendered and displayed in the monitor. The technology includes:

obtaining coordinate position of each voxel on each ultrasound beam and a distance between a voxel point and each channel of the ultrasound transducer, and calculating transmitting and receiving delays;

calculating the rotation angle of each voxel point in a spherical coordinate system, and performing 2D image beamforming;

after each rotation of the ultrasound transducer around the central axis by 360° (for a side-looking transducer) or 180° (for a forward-looking transducer), performing rapid 3D image reconstruction on all 2D images in the Cartier coordinate system according to the rotation angle,

when the four-dimensional imaging volumetric frame rate is set to f, the rotation speeds of the motor, the slip ring, and the transducer are f (for the side-looking transducer) or 0.5*f (for the forward-looking transducer);

performing interpolation according to the rotational angle, and calculating the gray value of each rotation angle based on the coordinate information; and

performing rendering and image displaying on the generated four-dimensional image.

In one embodiment, when the transducer unidirectionally and uniformly rotates at a high speed, the transducer transmits a plurality of focusing ultrasound beams, or transmits a plurality of non-focused plane waves or diverging waves, and receives reflected echo signals. Specifically, the transducer can transmit a plurality of focusing ultrasound beams, diverging waves or plane waves in sequence for 2D imaging.

In one embodiment, the transducer is connected to the catheter end connector through a wiring harness, and the wiring harness of the transducer is a coaxial cable or a flexible circuit board. The transducer rotates inside the sheath, and the sheath remains static.

In one embodiment, the wiring harness of the transducer, the transducer, and the torque coil are located in the sheath. During use, coupling liquid is injected into the proximal end of the catheter, and air between the ultrasound transducer and the sheath is exhausted.

In one embodiment, the wiring harness of the ultrasound transducer can be a coaxial cable or a flexible circuit board, which can be connected to a motor case directly or through the slip ring. The communication with the ultrasound console can be through a PCI-E interface, or another high-speed data transmission interface such as a USB or a network cable interface.

In one embodiment, the sheath is a deflectable sheath with gradually changing hardness. The distal end of the catheter is softer and the proximal end is harder. A sheath wall of the sheath is integrated with a pull wire, and the pull wire can be pulled through a handle to perform steering on the sheath.

Specifically, the coupling liquid needs to be filled outside the transducer and the catheter. A gap between the transducer and the catheter can be filled with the liquid by injecting the coupling liquid (which is usually normal saline, glycerol, or the like) for venting before intervention, namely in a manner of injecting the normal saline into the catheter for venting by using an injection syringe, or the coupling liquid can be sealed in the gap between the transducer and the catheter during production.

In the embodiments of the present disclosure, the extracorporeal motor (which may be a stepping motor, a servo motor, a linear motor, or the like), slip ring, and ultrasound console are all reusable components, and the catheter (including the sheath, the torque coil, the cable, the connector, and the ultrasound transducer) inserted into the body is a disposable consumable. During the intervention, the ICE catheter is first inserted into the cardiac chamber through peripheral venous intervention, and the angle of the catheter tip end is deflected through the handle to select a suitable imaging angle. Subsequently, the catheter retains fixed in position, and the motor is start rotation for 4D imaging.

FIG. 15 is a schematic diagram of a 4D ICE system with forward-looking rotational scanning. In this system, an ultrasound console is directly connected to a rotating case. A rotating motor and a slip ring are mounted inside the rotating case. A catheter is in a stationary state. The motor drives a transducer arranged in one dimension inside a front end of the catheter to perform unidirectional rotational imaging through a torque coil. In this solution, the transducer is placed inside the catheter, and the coupling liquid is filled between the transducer and the sheath for acoustic impedance matching.

As shown in FIG. 14, it is a schematic diagram of a 4D ICE system with side-looking rotational scanning. In this system, an ultrasound console is directly connected to a rotating case, and a rotating motor and a slip ring are arranged inside the rotating case. A catheter is in a stationary state. The motor drives a transducer arranged in one dimension inside a front end of the catheter to perform unidirectional rotational imaging through a torque coil. In this solution, the transducer is placed inside the catheter, and the coupling liquid is filled between the transducer and the sheath for acoustic impedance matching.

An embodiment of the present disclosure provides a forward-looking 4D ICE imaging system, including:

1. The motor and the multi-channel slip ring are located outside the body, and the motor drives the slip ring to rotate unidirectionally and uniformly through gear drive or a synchronous belt. The slip ring includes a stator side and a rotor side. The stator side is static and connected to the ultrasound console, while the rotor side is driven to rotate by the motor and is mechanically connected to a slip-ring-side connector. The slip ring can be reused. The catheter-side connector is connected to the ultrasound cable and the torque coil, and is disposable. During use, the catheter-side connector is connected to the console end connector.

2. During operation, the motor, the rotor side of the slip ring, and the slip-ring-side connector rotate uniformly and unidirectionally at N revolutions per second, thereby driving the catheter-side connector, the torque coil, the cable, and the ultrasound transducer to rotate uniformly and unidirectionally at N rps(revolutions per second). Meanwhile, the ultrasound console is electrically communicated to the ultrasound transducer through the stator side of the slip ring, the rotor side of the slip ring, the slip-ring-side connector, the catheter-side connector, and the cable.

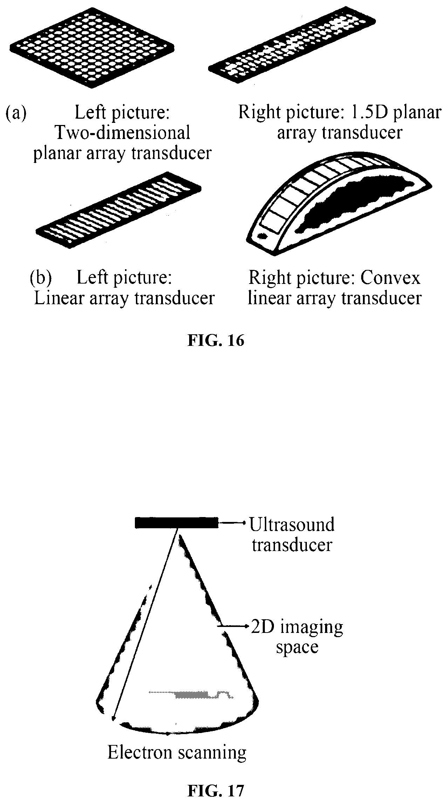

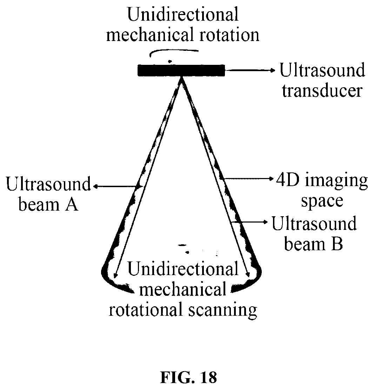

3. The transducer is a one-dimensional phased array transducer rotating around the rotation center. During imaging, the transducer rotates unidirectionally and uniformly at N rps, and performs 2D ultrasound imaging while rotating, that is, transmitting and receiving ultrasound beams. As shown in FIG. 18, during forward-looking imaging for every 180° rotation of the transducer, beam A and beam B rotate 180° to complete 360° scanning. That is, a complete three-dimensional scan of the space and a volumetric frame of 4D ICE image acquisition are completed. FIG. 17 shows a scanning method of a linear array forward-looking transducer, namely, a 2D imaging scanning method, without rotation. FIG. 18 shows a 3D scanning and imaging method during rotational imaging of a forward-looking transducer.

**4.** The ultrasound transducer emits ultrasound beams in different directions while rotating unidirectionally around the center. A four-dimensional image can be obtained by performing 3D reconstruction on the 2D images formed at different rotation angles and rendering the 2D images in the ultrasound console.

Assuming that the mechanical rotation is in an azimuth direction and ultrasound beam scanning is in an elevation direction, reconstruction steps are as follows:

(1) Assuming that there are M voxel points on each beam, a distance between the voxel points and the center of the transducer is denoted as $R_m$, m<MEN. Each voxel point is represented as $(A_m, E_m, R_m)$ in spherical coordinates, where $A_m$ and $E_m$ are rotation angles of the beam in the azimuth direction and the elevation direction.

(2) At every 180° rotation of the transducer, 3D reconstruction is performed once. In the reconstruction process, there are K voxels in a four-dimensional imaging space shown in FIG. 17, and a spatial position of each voxel is represented as $(x_k, y_k, z_k)$ in the Cartier coordinate system, where $k < K \in N$.

(3) 3D interpolation is performed. According to coordinate positions, there are various interpolation methods, including linear interpolation, polynomial interpolation, and other calculation methods, which will not be further explained in the embodiments of the present disclosure.

(4) 3D image rendering and displaying are performed. A 3D rendering algorithm, such as a ray-casting algorithm, is performed to render a 3D image on a display.

In the embodiments of the present disclosure, the ultrasound transducer implements imaging while rotating around its center as the rotation center. For the forward-looking four-dimensional ICE imaging scheme, when a clinically required imaging volumetric frame rate is 20 vol/s, through the embodiments of the present disclosure, the rotation speeds of the motor and the slip ring only need to reach 10 rps. In contrast, in the side-looking imaging scheme, the rotation speeds of the motor and the slip only need to reach 20 rps. Therefore, the forward-looking solution of the present disclosure greatly lowers the requirements for the motor and the slip ring. The transducer is in front view, which makes it easier to image regions of interest in the heart such as the left atrial appendage and the valves during the bending adjustment on the tip end of the catheter. In contrast, for systems such as Johnson NuVision also need to have a function of manually rotating the transducer inside the catheter to adjust an imaging field of view. In the present disclosure, manual rotation on the transducer is not required, and the motor can drive the transducer to automatically rotate.

An embodiment of the present disclosure provides 4D ICE imaging equipment with forward-looking or side-looking mechanical rotation. Compared with the existing commercial phased array 4D ICE technology, the array element rotating method described in this embodiment of the present disclosure can significantly reduce the equipment costs and the process difficulty. In another aspect, in the forward-looking solution in the present disclosure, when the transducer rotates a circle around the central axis, two frames of 4D images can be scanned, thus significantly improving the 4D imaging volumetric frame rate and lowering the requirement for a mechanical transmission system.

The embodiments of the present disclosure include the following advantages:

The forward-looking 4D ICE imaging system provided in the embodiments of the present disclosure at least includes: the ultrasound console, the slip ring, the connectors, the motor, the transducer, the sheath, and the torque coil. The ultrasound console is connected to the transducer sequentially through the slip ring, the connectors, the cable, and the torque coil. During use, the motor drives the multi-channel slip ring to rotate unidirectionally and uniformly, thereby driving the connectors, the torque coil, and the transducer to rotate uniformly and unidirectionally at a high speed within the sheath. Meanwhile, the transducer transmits the ultrasound beams in different directions and receives the reflected echoes to generate multiple frames of two-dimensional images rotating around the rotation center, and the two-dimensional images formed at different rotation angles are reconstructed to obtain four-dimensional images for rendering and real-time display.. Compared with the side-looking mechanical 4D ICE imaging, the transducer of the present disclosure is in front view and rotates around the central axis. Two 3D images can be obtained when the transducer rotates a circle. Therefore, this can reduce the rotation speed and lower the requirement for structures such as the motor, the slip ring, and a seal at the set four-dimensional imaging volumetric frame rate. The various embodiments in this specification are described in a progressive method, and each embodiment focuses on differences from other embodiments. The same or similar parts between all the embodiments can be referred to each other.

What is disclosed above is merely exemplary embodiments of the present disclosure, and certainly is not intended to limit the scope of the claims of the present disclosure. Therefore, equivalent variations made in accordance with the claims of the present disclosure shall fall within the scope of the present disclosure.

## Claims

1. A 4-dimensional (4D) intracardiac echocardiography (ICE) catheter, comprising:

   a sheath with an internal lumen;
   an ultrasound transducer assembly rotatably connected to the sheath and suspended in the internal lumen;
   a bearing, connected to the ultrasound transducer assembly and an external steering handle; and
   an external lumen connected to the bearing and located on one side of the bearing away from the ultrasound transducer assembly.

2. The 4D ICE catheter according to claim 1, wherein the ultrasound transducer assembly comprises an ultrasound transducer, a mounting rack, and a signal cable; the mounting rack is rotatably connected to the sheath; the ultrasound transducer is arranged on the mounting rack; and the signal cable is electrically connected to the ultrasound transducer and is configured to be connected to an external ultrasound console.

3. The 4D ICE catheter according to claim 2, wherein the sheath further internally comprises a coupling medium; the coupling medium fills the internal lumen and wraps around the ultrasound transducer assembly; and the coupling medium is a flexible medium.

4. The 4D ICE catheter according to claim 2, wherein the sheath comprises an acoustic housing and a supporting component; the supporting component is connected to the acoustic housing and encloses the internal lumen together with the acoustic housing; one end of the mounting rack is rotatably connected to the supporting component; the bearing comprises a bearing body and a sealing ring; the sealing ring is located on an inner side of the bearing body; the bearing body is connected to the acoustic housing and the steering handle; the steering handle is configured to deflect the acoustic housing; and the other end of the mounting rack is arranged to pass through the sealing ring and is connected to a drive unit on the steering handle.

5. The 4D ICE catheter according to claim 4, wherein the mounting rack comprises a first rotating shaft and a second rotating shaft; the first rotating shaft and the second rotating shaft are respectively disposed at two opposite ends of the mounting rack; the first rotating shaft is rotatably connected to the supporting component; and the second rotating shaft is rotatably connected to the sealing ring and is connected to the drive unit.

6. The 4D ICE catheter according to claim 4, wherein the bearing further comprises a torque coil respectively connected to the mounting rack and the drive unit;
   the torque coil is of a hollow structure; the signal cable is arranged to pass through the torque coil and is connected to the ultrasound connector; the ultrasound connector is connected to the ultrasound console; or, the torque coil is of a solid structure; and the signal cable is parallel to the torque coil.

7. The 4D ICE catheter according to claim 4, wherein the 4D ICE catheter further comprises a steering handle and the drive unit; the steering handle comprises a pull wire; the pull wire is embedded in the wall of the sheath and is individually connected to the steering handle and the bearing; the steering handle is configured to pull the pull wire thereby deflecting the sheath; the sheath is flexible and soft in the distal end, so that the distal end of the sheath deflects; the drive unit is connected to the ultrasound transducer assembly and is configured to drive the ultrasound transducer assembly to uniformly rotate within the sheath uni-directionally or bi-directionally.

8. Ultrasound imaging equipment, comprising an ultrasound console and the 4D ICE catheter according to claim 1.

9. The ultrasound imaging equipment according to claim 8, wherein the ultrasound imaging equipment has two imaging modes: a 2D imaging mode and a 4D imaging mode; when the bearing rotates slowly or remains stationary, the ultrasound imaging equipment is in 2D imaging mode; when the bearing rotates rapidly, the ultrasound imaging equipment is in the 4D imaging mode;

a position sensor is integrated in the ultrasound transducer of the ultrasound transducer assembly; when the bearing rotates slowly, the ultrasound imaging equipment can be synchronized by an electrocardiogram gating signal to perform image segmentation and 3D surface reconstruction on a series of 2D ultrasound images captured at different rotational positions at the cardiac end-diastolic phase, thus obtaining a 3D model of each specific cardiac structure at the cardiac end-diastolic phase; and
the image segmentation employs an artificial intelligence (AI)-based deep learning neural network method.

10. The ultrasound imaging equipment according to claim 8, wherein the ultrasound imaging equipment further comprises an mechanical driving device;

the mechanical driving device is arranged at a proximal end of the 4D ICE catheter; the mechanical driving device and the ultrasound console are respectively connected to the ultrasound transducer assembly;
the ultrasound console is configured to output a acoustic wave driving signal;
the ultrasound transducer assembly is configured to intermittently emit the acoustic wave driving signal outputted by the ultrasound console;
the mechanical driving device is configured to: rotate, when the ultrasound transducer assembly emits the acoustic wave driving signal activated by the ultrasound console, the ultrasound transducer assembly to rotate uniformly within the 4D ICE catheter;
the ultrasound transducer assembly is further configured to: receive an activation signal outputted by the ultrasound console, and transmit the reflected echo signal to the ultrasound console;
and the ultrasound console is further configured to perform signal post-processing on the received echo signal to generate a 4D ultrasound image of the imaging target.

11. The ultrasound imaging equipment according to claim 10, wherein the mechanical driving device comprises a motor, a multi-channel slip ring, and a console end connector; the motor is fixedly connected to a stator of the multi-channel slip ring; the console end connector is fixedly connected to a rotor of the multi-channel slip ring; the console end connector is connected to a catheter-side connector; the motor drives the rotor of the multi-channel slip ring to rotate through mechanical transmission; and the motor, the multi-channel slip ring, and the console end connector are all reusable.

12. The ultrasound imaging equipment according to claim 10, wherein the ultrasound transducer assembly comprises an ultrasound transducer; the ultrasound transducer is a phased array transducer for 2D imaging, or a plurality of phased array transducers for 2D imaging, which are combined at a preset rotation angle;
the ultrasound transducer comprises a transducer and an acoustic lens; and the acoustic lens focuses ultrasonic waves emitted by the transducer, to enhance a signal-to-noise ratio of ultrasonic waves at a focused position.

13. The ultrasound imaging equipment according to claim 8, wherein the ultrasound imaging equipment further comprises a slip ring, a console end connector, a catheter end connector, a motor, and a torque coil; the ultrasound console is connected to the console end connector through the slip ring; the catheter end connector is connected to the ultrasound transducer assembly through a cable and the torque coil;

the motor is configured to drive a rotor side of the slip ring to rotate uniformly at a high speed, and is also configured to drive the rotor side of the slip ring to rotate slowly to achieve 3D modeling of a heart in the end-diastolic phase; the slip ring comprises the rotor side and a stator side; the stator side is directly connected to the console end connector; the rotor side is connected to the catheter; the motor drives the rotor side of the slip ring to rotate, thus driving the rotor-side connector, the torque coil, and the ultrasound transducer assembly to rotate around a center of the ultrasound transducer assembly in the sheath;
the torque coil is configured to drive the ultrasound transducer assembly to rotate within the sheath;
the ultrasound transducer assembly is configured to transmit and receive ultrasound beams in different directions;

the ultrasound console is configured to: generate, based on ultrasound focused beams or plane waves in different directions, 2D images that rotate at a high speed around a central axis, perform 3D reconstruction on 2D images formed at different rotation angles, and perform rendering and image displaying on a reconstructed 4D image; the ultrasound transducer assembly performs forward-looking or side-looking imaging; during side-looking imaging, the ultrasound transducer assembly reconstructs a volume of 4D image at every 360° rotation; when an ultrasound 4D imaging volumetric frame rate is set to f, rotation speeds of the motor and the slip ring are f; during forward-looking imaging the ultrasound transducer assembly reconstructs a volume of 4D image at every 180° rotation; and when the ultrasound 4D imaging volumetric frame rate is set to f, rotation speeds of the motor and the slip ring are 0.5*f.

14. The ultrasound imaging equipment according to claim 13, wherein the ultrasound console emits and receives the ultrasound beams in different directions through the ultrasound transducer assembly, generates a plurality of frames of 2D images rotating around a center of a rotating shaft, performs rapid 3D reconstruction on the 2D images formed at different rotation angles, and renders and displays the reconstructed 4D image, comprising:

obtaining coordinate position of each voxel on each ultrasound beam and a distance between a voxel and each channel of the ultrasound transducer, and calculating transmitting and receiving delays;
calculating a rotation angle of each voxel point in a spherical coordinate system, and performing 2D image beamforming;
after the ultrasound transducer assembly rotates around the central axis 180° forward-looking rotation or 360° side-looking each time, performing 3D image reconstruction on all the 2D images in a Cartier coordinate system based on the rotation angle, wherein when the 4D imaging volumetric frame rate is set to f, the rotation speeds of the motor, the slip ring, and the transducer are 0.5*f for forward-looking or f for side-looking;
performing interpolation processing according to the rotation angle, and calculating a gray value of each rotation angle based on coordinate information; and
performing rendering and image displaying on the generated 4D image.

15. An ultrasound imaging method, wherein the ultrasound imaging equipment according to claim 9 is used, and the ultrasound imaging method comprises the following steps:

exciting the ultrasound transducer through a signal cable and driving the ultrasound transducer to rotate within the static sheath, transmitting acoustic beam in different directions sequentially, and receiving echo beams;
filtering noise in the ultrasound console;
obtaining the spherical coordinates of received echo beams based on the rotation speed and flight time of the echo;
reconstructing a 4D image based on the receiving echo beams.

<u>10</u>

**FIG. 1**

**FIG. 2**

<u>1</u>

**FIG. 3**

210 ⌐ 100

D

R

S

10

**FIG. 4**

Excite an ultrasound transducer through a signal cable and drive the ultrasound transducer to rotate within a static sheath, transmit acoustic beams at different angles sequentially, and receive echo beams — S100

Filter noise in an ultrasound console — S200

Obtain spherical coordinates of the received echo beams based on a rotation speed and flight time of the echo — S300

Conduct beamforming to obtain a series of 2D images at different rotation angles, followed by reconstructing a 3D image based on the 2D images — S400

Use advance artificial intelligence to segment key anatomies of heart in both 2D and 3D — S500

**FIG. 5**

**FIG. 6**

**FIG. 7**

FIG. 8

(a)

(b)          (c)          (d)

FIG. 9

An echo signal is acquired, where the echo signal is that, when a mechanical driving apparatus drives a miniature ultrasound transducer to rotate uniformly and unidirectionally, the miniature ultrasound transducer emits an acoustic wave driving signal intermittently and receives an echo signal | 401

The echo signal is subjected to signal post-processing, including filtering and beamforming, to obtain a beamformed image. | 402

Spherical coordinates of each voxel point of the beamformed image are acquired according to a rotation speed of the mechanical driving apparatus and time of receiving the echo signal. | 403

The voxel points expressed in the spherical coordinates are interpolated by a preset interpolation algorithm, and the spherical coordinates of the voxel points in a spherical coordinate system aretransformed into image pixels in Cartier coordinate system to obtain a 4D ultrasound image of animaging target composed of image pixels. | 404

**FIG. 10**

**FIG. 11**

/601 Signal acquisition module — /602 Beamforming module — /603 Coordinate calculation module — /604 Coordinate transformation module

**FIG. 12**

Processor

System bus

Operating system

Computer program

Non-volatile storage medium

Computer program

Internal memory

Network interface

Computer device

**FIG. 13**

Unidirectional
rotation

Imaging
field of view

Catheter

One-dimensional
transducer

Rotation cavity

Spring

Wiring hardness
of transducer

Ultrasound console

Slip ring

Rotating
motor

**FIG. 14**

Imaging
field of view

Catheter

One-dimensiona
transducer

Rotation cavity

Unidirectional
rotation

Spring

Wiring
hardness
of transducer

Ultrasound console

Slip ring

Rotating motor

**FIG. 15**

(a)    Left picture:        Right picture: 1.5D planar
Two-dimensional        array transducer
planar array transducer

(b)   Left picture:       Right picture: Convex
Linear array transducer   linear array transducer

**FIG. 16**

Ultrasound
transducer

2D imaging
space

Electron scanning

**FIG. 17**

FIG. 18

Europäisches
Patentamt

European
Patent Office

Office européen
des brevets

## EUROPEAN SEARCH REPORT

Application Number

EP 25 22 0500

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 117 064 448 A (SHANGHAI MICROPORT EP MEDTECH CO LTD) 17 November 2023 (2023-11-17) | 1-3,8, 10,12 | INV. A61B8/08 A61B8/12 |
| Y | * paragraphs [0002] - [0045]; figures 1-7 * ----- | 4-7,9, 11,13-15 | A61B8/00 |
| Y | PROULX T.L. ET AL: "Advances in catheter-based ultrasound imaging Intracardiac Echocardiography and the ACUSON AcuNavTM Ultrasound Catheter", ULTRASONICS SYMPOSIUM, 2005 IEEE ROTTERDAM, THE NETHERLANDS 18-21 SEPT. 2005 PISCATAWAY, NJ, USA,IEEE, vol. 1, 18 September 2005 (2005-09-18), pages 669-678, XP010898829, DOI: 10.1109/ULTSYM.2005.1602941 ISBN: 9780780393820 * page 670, right-hand column, paragraph 3 - page 671, left-hand column, paragraph 4; figures 4,6 * * side-viewing and forward-viewing; page 674, right-hand column, paragraph 1 - page 675, left-hand column, paragraph 1; figures 11,12a-b,13 * ----- | 4-7,9, 13-15 | |
| Y | US 2015/366536 A1 (COURTNEY BRIAN [CA] ET AL) 24 December 2015 (2015-12-24) * paragraph [0075]; figures 3c-d * ----- -/-- | 11 | |

TECHNICAL FIELDS SEARCHED (IPC)

A61B

The present search report has been drawn up for all claims

| Place of search | Date of completion of the search | Examiner |
|---|---|---|
| Munich | 2 April 2026 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.................................................................................................
& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 25 22 0500

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BELOHLAVEK ET AL: "Toroidal geometry: Novel three-dimensional intracardiac imaging with a phased-array transducer", JOURNAL OF THE AMERICAN SOCIETY OF ECHOCARDIOGRAPHY. MOSBY-YEAR BOOK, INC. ST. LOUIS, MO, US, vol. 10, no. 5, 1 June 1997 (1997-06-01), pages 493-498, XP005699789, ISSN: 0894-7317, DOI: 10.1016/S0894-7317(97)70001-4 * abstract; figure 1 * * page 494, right-hand column, paragraph 2 * ----- | 14 | |
| X | CN 117 481 696 A (SHENZHEN XINHUAN TECH CO LTD) 2 February 2024 (2024-02-02) | 1,8 | |
| A | * paragraphs [0003] - [0066]; figures 1,2 * ----- | 3,4,6,7, 10-14 | |
| | | | **TECHNICAL FIELDS SEARCHED (IPC)** |

The present search report has been drawn up for all claims

| Place of search | Date of completion of the search | Examiner |
|---|---|---|
| Munich | 2 April 2026 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 22 0500

This annex lists the patent family members relating to the patent documents cited in the above-mentioned European search report.
The members are as contained in the European Patent Office EDP file on
The European Patent Office is in no way liable for these particulars which are merely given for the purpose of information.

02-04-2026

| Patent document cited in search report | | Publication date | Patent family member(s) | | Publication date |
|---|---|---|---|---|---|
| CN 117064448 | A | 17-11-2023 | NONE | | |
| US 2015366536 | A1 | 24-12-2015 | CA | 2815580 A1 | 18-05-2012 |
| | | | CA | 3085777 A1 | 18-05-2012 |
| | | | EP | 2637555 A1 | 18-09-2013 |
| | | | EP | 3988011 A1 | 27-04-2022 |
| | | | JP | 6371421 B2 | 08-08-2018 |
| | | | JP | 6673985 B2 | 01-04-2020 |
| | | | JP | 7069236 B2 | 17-05-2022 |
| | | | JP | 7627243 B2 | 07-02-2025 |
| | | | JP | 2013541392 A | 14-11-2013 |
| | | | JP | 2017113576 A | 29-06-2017 |
| | | | JP | 2019000651 A | 10-01-2019 |
| | | | JP | 2020103935 A | 09-07-2020 |
| | | | JP | 2022106900 A | 20-07-2022 |
| | | | US | RE49218 E | 27-09-2022 |
| | | | US | RE50513 E | 05-08-2025 |
| | | | US | 2013216114 A1 | 22-08-2013 |
| | | | US | 2015366536 A1 | 24-12-2015 |
| | | | WO | 2012061940 A1 | 18-05-2012 |
| CN 117481696 | A | 02-02-2024 | NONE | | |

EPO FORM P0459

For more details about this annex : see Official Journal of the European Patent Office, No. 12/82